# EUROPÄISCHE PATENTSCHRIFT

(11) **EP 0 701 820 B1**
(45) Veröffentlichungstag und Bekanntmachung des Hinweises auf die Patenterteilung: **20.01.1999**
(21) Anmeldenummer: 95106348.6
(22) Anmeldetag: 27.04.1995
(51) Int. Cl.: A61L 2/18, A61L 2/04

(54) **Verfahren zum Reinigen und Desinfizieren von Apparaten und Instrumenten**
Method for cleaning and disinfecting of apparatus and instruments
Procédé de nettoyage et de désinfection d'appareils et d'instruments

(30) Priorität: 16.09.1994 DE 4433137
(43) Veröffentlichungstag der Anmeldung: 20.03.1996
(73) Patentinhaber: CHEMISCHE FABRIK DR. WEIGERT (GMBH & CO.), 20539 Hamburg (DE)
(72) Erfinder: Staffeldt, Jürgen, Dr., D-21423 Winsen/Luhe (DE)
(74) Vertreter: Glawe, Delfs, Moll & Partner

(56) Entgegenhaltungen:
- DE-A- 2 749 448
- DE-A- 2 852 072
- DE-A- 3 601 744

## Beschreibung

Die Erfindung betrifft ein Verfahren zum maschinellen Reinigen und thermischen Desinfizieren von medizinischen und biotechnologischen Apparaten und Instrumenten unter thermischer Desinfektion der Reinigerflotte in einer Eintankmaschine.

Für die Reinigung und Desinfektion von medizinischen oder chirurgischen sowie biotechnologischen Instrumenten und Apparaturen werden Spezialspülmaschinen eingesetzt; die Desinfektion erfolgt thermisch. Als Spülmaschinen werden hier im wesentlichen zwei Arten von Maschinen eingesetzt, und zwar einerseits sogenannte Eintankmaschinen mit Frischwasserwechsel, z. B. gemäß DE-A-36 01 777 und DE-A-33 27 466, und andererseits sogenannte Taktstraßen, in denen die Instrumente nacheinander verschiedene Reinigungs- und Desinfektionszonen durchlaufen.

Der Programmablauf bei den Eintankmaschinen ist typischerweise wie folgt: Kalt- oder Warmwasser läuft in die Maschine ein und wird mit einem alkalischen Reiniger versetzt. Diese alkalische Reinigungslösung wird anschließend auf Thermodesinfektionstemperaturen von 93°C aufgeheizt. Nach einer Temperaturhaltezeit von z.B. 10 min erfolgt das Abpumpen der heißen alkalischen Lauge; anschließend erfolgt eine Neutralisation mit einem sauren Reagens sowie eine Nachspülung mit Wasser in mehreren Nachspülgängen. Taktstraßenmaschinen beginnen aus Gründen der besseren Reinigungswirkung mit einer warmen, sauren Vorreinigung. In einem nachfolgenden Tank erfolgt eine alkalische Hauptreinigung und die thermische Desinfektion; die üblichen Spülgänge schließen sich an.

Speziell bei Apparaten und Instrumenten, die mit Rückständen von Körperflüssigkeiten verunreinigt sind, wird im allgemeinen mit einer sauren Reinigung ein besseres Reinigungsergebnis erzielt. Ein saurer Reiniger ist jedoch in Eintankmaschinen mit Frischwasserwechsel, wie sie oben beschrieben wurden, nicht einsetzbar, weil eine Thermodesinfektion mit einer sauren Reinigungslösung erhebliche Materialschäden an den Instrumenten verursacht.

Eine kalte oder warme saure Vorreinigung mit anschließendem Abpumpen der dabei verwendeten sauren Reinigungslösung, gefolgt von einem zweiten Reinigungsschritt mit einem alkalischen Reiniger und einer thermischen Desinfektion, ist in diesen Maschinen jedoch ebenfalls nicht anwendbar, da derartige Maschinen vom Bundesgesundheitsamt (BGA) nicht zugelassen werden. Die BGA-Zulassung hat zur Voraussetzung, daß im ersten Behandlungsschritt in der Maschine bereits die Thermodesinfektion erfolgen muß, d.h. es muß bereits das erste, aus der Maschine ablaufende Abwasser desinfiziert sein.

Somit war es bisher nicht möglich, in einer Eintankmaschine mit Frischwasserwechsel einer handelsüblichen Art, z. B. eines von der Firma Miele, Gütersloh, hergestellten Typs, eine saure Vorreinigung, wie sich diese z. B. bei Laborglas mit Blutrückständen bewährt hat, durchzuführen.

Da jedoch saure Reiniger im Vergleich zu alkalischen Reinigern verbesserte Reinigungsergebnisse liefern, besteht ein Bedürfnis an Verfahren für Eintankmaschinen, in denen die erste Reinigung im sauren Milieu erfolgt.

Dieses Ziel läßt sich erfindungsgemäß dadurch erreichen, daß man in Eintankmaschinen der handelsüblichen Art ein Verfahren einsetzt, gemäß dem man die Apparate und Instrumente mit einer sauren bzw. sauer eingestellten Reinigerflotte bei Temperaturen bis zu 55°C behandelt, der Reinigerflotte in Gegenwart der Apparate und Instrumente ein mit dieser verträgliches basisches Reinigungs- und Neutralisationsmittel unter Einstellung eines PH-Wertes von mindestens 6,5 zusetzt und die neutralisierte bzw. alkalisierte Reinigerflotte sowie die mit der Reinigerflotte in Kontakt befindlichen Apparate und Instrumente thermischen Desinfektionsbedingungen unterwirft.

Gemäß dem Verfahren der Erfindung wird somit eine Eintankmaschine der üblichen Art mit Frischwasserwechsel mit Kaltwasser befüllt. Dem kalten Wasser wird ein sauer Reiniger zudosiert; das sauer eingestellte Reinigungsmedium wird anschließend für die Reinigung in einem relativ niedrigen Temperaturbereich benutzt. Damit lassen sich Beschädigungen an Instrumenten, insbesondere durch Korrosion, ausschalten. Nach einer gewissen Einwirk- bzw. Reinigungszeit, wobei die dabei verwendete kalte, saure Reinigungslösung auch allmählich auf Temperaturen bis zu 55°C, insbesondere bis zu 40°C, erwärmt werden kann, erfolgt die Zudosierung eines alkalischen Reinigungs- und Neutralisationsmittels zur Erzeugung der für die Thermodesinfektion aus Korrosionsschutzgründen erforderlichen alkalischen Reinigungslösung. Der Reiniger muß dabei so beschaffen sein, daß er beim Zudosieren in die saure Reinigungslösung nicht zu Ausflockungen oder Ausfällungen führt. Dabei wird neutralisiert, bis der pH-Wert mindestens 6,5 und bis zu 7,5 beträgt, oder es wird insbesondere überneutralisiert, bis der pH-Wert der Reinigerlösung im Bereich von 9 bis 10 liegt. Anschließend folgt ein weiteres Aufheizen bis zur Thermodesinfektionstemperatur. Anschließend wird wie üblich eine Reinigungszeit von z. B. 10 min eingehalten; die üblichen Neutralisations- und Nachspülgänge schließen sich an. Die vorerwähnten Zeiten können im übrigen - je nach Bedarf - verkürzt oder verlängert werden, solange das angestrebte Ergebnis der Desinfektion von Instrumenten und Reinigerflotte hierdurch nicht beeinträchtigt wird. Das Verfahren der Erfindung hat den weiteren Vorteil, daß die Reinigung der zu behandelnden Apparate und Instrumente einmal im sauren Milieu und zum anderen im neutralen bzw. alkalischen Milieu erfolgt, so daß auch solche Verunreinigungen wirksam entfernt werden, die sich nur im Sauren oder nur im Neutralen bis Alkalischen besonders gründlich entfernen lassen.

Gemäß einer bevorzugten Ausführungsform weist die Reinigerflotte einen pH-Wert im Bereich von 1 bis 4 auf; die Behandlung mit der sauren Reinigerflotte erfolgt vorzugsweise im Temperaturbereich zwischen 10 und 30°C, insbesondere bei Umgebungstemperatur.

Gemäß einer weiteren vorteilhaften Ausführungsform der Erfindung weist die Reinigerflotte nach der Neutralisation bzw. der Alkalisierung einen pH-Wert im Bereich von 7 bis 12, insbesondere von 9 bis 12, auf.

Gemäß einer weiteren vorteilhaften Ausführungsform der Erfindung erfolgt die thermische Desinfektion bei Temperaturen oberhalb von 90°C, wobei die Dauer der thermischen Desinfektion vorzugsweise mindestens 10 min beträgt. Es handelt sich hierbei um für Deutschland empfohlene Parameter. Es sind jedoch auch nach den jeweiligen nationalen Vorschriften andere Betriebsparameter für die thermische Desinfektion möglich, z. B. 85°C und 3 Minuten.

Nach Beendigung der thermischen Desinfektion wird die Reinigerlösung abgepumpt und die Reinigungsanlage mit Frischwasser beschickt. Diesem Frischwasser wird, falls bei der thermischen Desinfektion alkalisch gearbeitet wurde, ein Neutralisationsmittel zugesetzt, vorzugsweise die in der ersten Stufe eingesetzte saure Reinigerlösung. Es ist auch möglich, die saure Reinigerlösung direkt in die alkalische Lösung nach der thermischen Desinfektion zur Neutralisation derselben einzudosieren. Das Verfahren wird durch mindestens einen Spülgang mit Frischwasser, vorzugsweise gefolgt von einem Spülgang mit erwärmtem, entmineralisiertem Wasser und den üblichen thermischen Trocknungsstufen, abgeschlossen.

Nachfolgend werden bevorzugte Ausführungsbeispiele für die Durchführung des Verfahrens der Erfindung gegeben.

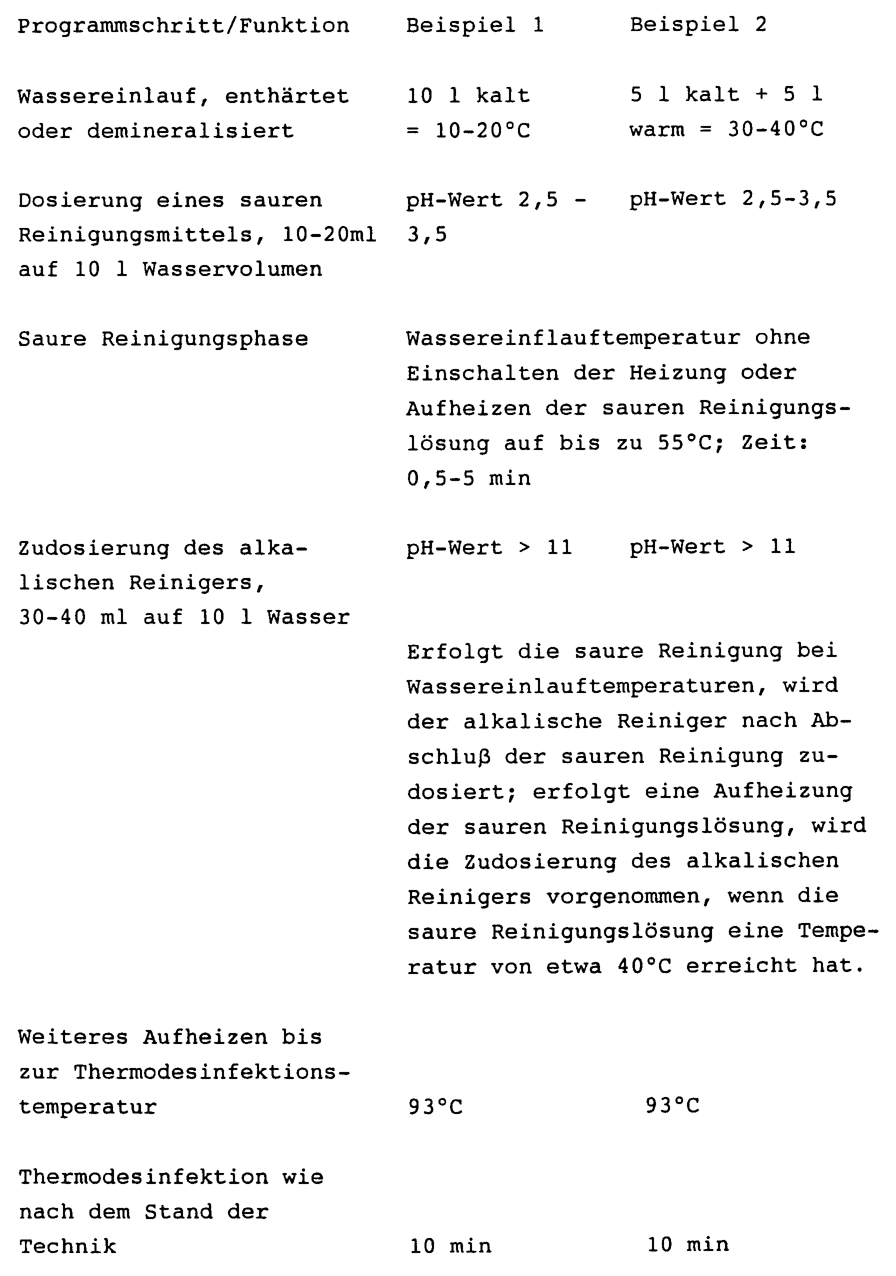

Typische Beispiele für in dem Verfahren der Erfindung einzusetzende saure Reiniger sind organische Monocarbonsäuren wie Ameisensäure oder Essigsäure oder Hydroxycarbonsäuren wie Zitronensäure oder Äpfelsäure. Auch anorganische Säuren wie Phosphorsäure, Schwefelsäure, Salpetersäure oder Amidosulfonsäure sind möglich, weiterhin können auch Persäuren, z. B. Peressigsäure, eingesetzt werden.

Der saure Reiniger kann außer den oben beschriebenen Säuren Hilfsstoffe wie Dispergatoren, Netzmittel, korrosionsverhindernde Zusätze oder dergleichen enthalten.

Der in dem Verfahren der Erfindung einsetzbare alkalische Reiniger kann z. B. Kaliumhydroxid, Natriumhydroxid, Nitrilotrisessigsäure, EDTA (Ethylendiamintetraessigsäure), Polyphosphat, Härtedispergatoren, Tenside sowie korrosionsverringernde Zusätze, z. B. Aminderivate wie Triethanolamin enthalten. Die vorerwähnten Inhaltsstoffe sind an sich nicht neu und sind dem Reinigungsfachmann auf dem hier angesprochenen Gebiet bestens bekannt.

Eine besonders bevorzugte Rezeptur für den sauren und den alkalischen Reiniger zur Verwendung in dem Verfahren der Erfindung wird im folgenden wiedergegeben.
- Saurer Reiniger:: 62 % Phosphorsäure
2 % Zitronensäure
Rest: vollentsalztes Wasser
- Alkalischer Reiniger:: 14 % Natriumhydroxid
16 % Nitrilotrisessigsäure
1,5 % Tensid (Natriumalkylaminodipropionat)
- Rest:: vollentsalztes Wasser

Die vorgenannten Prozentzahlen beziehen sich auf Gew.-%.

### Beispiel 3:

Es wurde eine handelsübliche Eintankmaschine vom Typ Miele G 7735 OP zur Instrumentenaufbereitung mit Mikroprozessorsteuerung verwendet. Der Programmablauf war wie folgt:

In die Spülmaschine wurden 10 l kaltes enthärtetes Wasser eingegeben und mit 10 ml eines sauren Reinigers mit 39 Gew.-% Zitronensäure und 1 Gew.-% Äpfelsäure versetzt. Die resultierende saure Reinigungslösung wurde von der Umwälzpumpe der Spülmaschine 2 min lang für eine saure Reinigung chirurgischer Instrumente verwendet. Anschließend wurde die saure Reinigungslösung durch automatisches Einschalten der Heizung auf 40°C aufgeheitzt. Hierfür wurde eine Zeit von 4 min benötigt. Anschließend erfolgte eine Zudosierung von 30 ml eines alkalischen Reinigers. Der alkalische Reiniger enthielt 14 % Natriumhydroxid, 16 % NTA sowie 1,5 % Natriumalkylaminodipropionat (handelsüblich). Nach Zudosierung des alkalischen Reinigers wies die Flotte einen pH-Wert von 11 auf. Ausflockungen in der nun alkalisierten Flotte wurden nicht beobachtet. Während der Zudosierung des alkalischen Reinigers und nach Beendigung der Dosierung wurde weiter aufgeheizt, bis eine Thermodesinfektionstemperatur von 93°C erreicht war. Von Beginn der Dosierung des alkalischen Reinigers bei 40°C bis zum Erreichen der Temperatur von 93°C vergingen 7 min. Nach dem Erreichen von 93°C erfolgte die übliche und bekannte Thermodesinfektion sowie der dann anschließende übliche und bekannte Programmablauf des Nachspülens und Trocknens.

## Patentansprüche

1. Verfahren zum maschinellen Reinigen und thermischen Desinfizieren von medizinischen und biotechnologischen Apparaten und Instrumenten mit thermischer Desinfektion der Reinigerflotte in einer Eintankmaschine, dadurch gekennzeichnet, daß man die Apparate und Instrumente mit einer sauren bzw. sauer eingestellten Reinigerflotte bei Temperaturen bis zu 55°C behandelt, der Reinigerflotte in Gegenwart der Apparate und Instrumente ein mit dieser verträgliches basisches Reinigungs- und Neutralisationsmittel unter Einstellung eines pH-Wertes von mindestens 6,5 zusetzt und die neutralisierte bzw. alkalisierte Reinigerflotte sowie die mit der Reinigerflotte in Kontakt befindlichen Apparate und Instrumente unter Erhitzen desinfiziert.

2. Verfahren nach Anspruch 1, dadurch gekennzeichnet, daß die Reinigerflotte einen pH-Wert im Bereich von 1 bis 4 aufweist.

3. Verfahren nach Anspruch 1 oder 2, dadurch gekennzeichnet, daß die Behandlung mit der sauren Reinigerflottte im Temperaturbereich zwischen 10 und 30°C, insbesondere bei Umgebungstemperatur erfolgt.

4. Verfahren nach mindestens einem der Ansprüche 1 bis 3, dadurch gekennzeichnet, daß die Reinigerflotte nach der Neutralisation bzw. der Alkalisierung einen pH-Wert im Bereich von 7 bis 12 aufweist.

5. Verfahren nach mindestens einem der Ansprüche 1 bis 4, dadurch gekennzeichnet, daß die thermische Desinfektion bei Temperaturen über 84°C, insbesondere über 90°C erfolgt.

6. Verfahren nach Anspruch 5, dadurch gekennzeichnet, daß die thermische Desinfektion über mindestens 3 min, insbesondere mindestens 10 min erfolgt.

## Claims

1. A method for the mechanical cleaning and thermal disinfection of medical and biotechnological apparatus and instruments with thermal disinfection of the cleansing liquor in a single-tank machine, characterised in that the apparatus and instruments are treated with an acid or acidified cleansing liquor at temperatures of up to 55°C, in the presence of the apparatus and instruments a compatible basic cleansing and neutralizing agent is added to the cleansing liquor, the pH being adjusted to a value of at least 6.5 and the neutralized and/or basified cleansing liquor and the apparatus and instruments in contact with the cleansing liquor are disinfected while being heated.

2. A method according to Claim 1, characterized in that the cleansing liquor has a pH value in the range of from 1 to 4.

3. A method according to Claim 1 or 2, characterised in that the treatment with the acid cleansing liquor takes place in the temperature range of between 10 and 30°C, in particular at ambient temperature.

4. A method according to at least one of Claims 1 to 3, characterised in that, after the neutralization and/or basification, the cleansing liquor has a pH value in the range of from 7 to 12.

5. A method according to at least one of Claims 1 to 4, characterised in that the thermal disinfection takes place at temperatures above 84°C, in particular above 90°C.

6. A method according to Claim 5, characterised in that the thermal disinfection takes place over at least 3 min., in particular at least 10 min.

## Revendications

1. Procédé de nettoyage mécanique et de désinfection thermique d'appareils et instruments médicaux et biotechnologiques, avec désinfection thermique du bain d'épuration dans une machine formée d'une seule cuve, caractérisé en ce que les appareils et instruments sont traités dans un bain d'épuration acide ou à réglage acide à des températures allant jusqu'à 55 °C, en ce qu'un agent de nettoyage et de neutralisation basique, compatible avec ledit bain d'épuration est ajouté dans le bain d'épuration contenant les appareils et instruments en régulant une valeur du pH de 6,5 au moins, et en ce que le bain d'épuration neutralisé ou alcalinisé, ainsi que les appareils et instruments en contact avec le bain d'épuration sont désinfectés à la chaleur.

2. Procédé selon la revendication 1, caractérisé en ce que le bain d'épuration présente une valeur de pH comprise entre 1 et 4.

3. Procédé selon la revendication 1 ou 2, caractérisé en ce que le traitement avec le bain d'épuration acide est effectué dans la plage de température comprise entre 10 et 30 °C, en particulier à température ambiante.

4. Procédé selon au moins l'une quelconque des revendications 1 à 3, caractérisé en ce que le bain d'épuration présente une valeur de pH comprise entre 7 et 12 après la neutralisation ou l'alcalinisation.

5. Procédé selon au moins l'une quelconque des revendications 1 à 4, caractérisé en ce que la désinfection thermique est exécutée à des températures supérieures à 84 °C, en particulier supérieures à 90 °C.

6. Procédé selon la revendication 5, caractérisé en ce que la désinfection thermique est exécutée pendant une durée d'au moins 3 mn, en particulier d'au moins 10 mn.
